# NEUE EUROPÄISCHE PATENTSCHRIFT

(11) **EP 2 941 480 B2**
(45) Veröffentlichungstag und Bekanntmachung des Hinweises auf die Entscheidung über den Einspruch: **24.07.2019**
(45) Hinweis auf die Patenterteilung: 17.08.2016
(21) Anmeldenummer: 13810891.5
(22) Anmeldetag: 11.12.2013
(51) Int. Cl.: C12M 1/12, C12M 3/00, C12M 1/00, B01L 7/00, B01L 9/00

(54) **INKUBATOR**
INCUBATOR
INCUBATEUR

(30) Priorität: 07.01.2013 DE 102013000044
(43) Veröffentlichungstag der Anmeldung: 11.11.2015
(73) Patentinhaber: Inheco Industrial Heating and Cooling GmbH, 82152 Martinsried (DE)
(72) Erfinder: MOMBOISSE, Michael, 81669 München (DE); GEORGE, Christian, 82515 Wolfratshausen (DE); LOB, Volker, 81375 München (DE)
(74) Vertreter: Grättinger Möhring von Poschinger Patentanwälte Partnerschaft
(86) Internationale Anmeldenummer: PCT/EP2013/003735
(87) Internationale Veröffentlichungsnummer: WO 2014/106526

(56) Entgegenhaltungen:
- WO-A1-2012/141055
- DE-A1-102005 036 763
- FR-A1- 2 849 862
- FR-A1- 2 849 862
- JP-A- 2004 267 117
- US-A1- 2004 152 188
- US-A1- 2005 051 723

## Beschreibung

Die vorliegende Erfindung betrifft einen Inkubator zur Beaufschlagung von in Probenbehältnissen aufgenommenen Proben mit einer spezifisch vorgebbaren Temperatur umfassend eine temperierbare Probenkammer mit wenigstens einer verschließbaren Zugangsöffnung und wenigstens eine einen Probenbehältnisträger tragende Ein- und Ausfuhrmechanik, mit welcher der seinerseits ein Probenbehältnis tragende Probenbehältnisträger durch eine Zugangsöffnung des Inkubators in die Probenkammer eingefahren und aus dieser herausgefahren werden kann.

Inkubatoren der vorstehend genannten Art sind aus dem Stand der Technik, z.B. aus der DE 10 2005 036 763 A1, hinlänglich bekannt. Zum Be- und Entladen der Probenkammer dient dabei eine das Probenbehältnis tragende Schublade und eine entsprechende Schubladenmechanik vorgesehen, mit welcher das Probenbehältnis durch eine mittels eines Deckels verschließbare Zugangsöffnung des Inkubators in die Probenkammer eingefahren und nach Durchführung eines Inkubationsvorgangs wieder aus der Probenkammer herausgefahren werden kann. Die die Schublade und den Deckel tragende Schubladenmechanik ist dabei zumindest teilweise innerhalb der Probenkammer angeordnet und montiert.

Aufgrund der zumindest teilweise gegebenen Anordnung und Montage der Schubladenmechanik innerhalb der Probenkammer wird die Schubladenmechanik eines solchen Inkubators häufig der gegebenenfalls schädlichen Atmosphäre in der Probenkammer ausgesetzt, die insbesondere durch innerhalb des Inkubators ggfs. teilweise verdampfende chemische oder biologische Proben oder durch extern in die Probenkammer eingeleitete Gase verunreinigt ist, was zum eine regelmäßige Reinigung der Schubladenmechanik erforderlich macht und zum anderen - z.B. aufgrund von hierdurch beförderten Korrosionsprozessen - nachteilig für die Lebensdauer einer entsprechenden Schubladenmechanik ist. Ferner ist hierdurch auch die Probenkammer als solche wegen der zumindest teilweise darin angeordneten bzw. montierten Schubladenmechanik schwer und umständlich zu reinigen, was sich ebenfalls als nachteilig erweist.

Des Weiteren sind aus dem Stand der Technik auch (externe) Robotersysteme bekannt, mit welchen ein Probenbehältnis (z.B. eine Mikrotiterplatte zur gleichzeitigen Aufnahme einer Mehrzahl an Proben) in die Probenkammer eines Inkubators eingefahren, dort abgesetzt und nach Abschluss des Inkubationsvorgangs wieder angehoben und aus dieser herausgefahren werden kann. Dabei ist stets vorgesehen, dass das als Ein- und Ausfuhrmechanik dienende Robotersystem bei geöffneter Zugangsöffnung in die Probenkammer hineinreicht und vor dem Schließen der Zugangsöffnung wieder aus der Probenkammer herausgefahren werden muss, was auch in zeitlicher Hinsicht nachteilig ist. Ferner sind handelsübliche Robotersysteme in der Regel nicht zum Absetzen oder Aufnehmen von Probenbehältnissen in einer räumlich beengten Probenkammer geeignet und müssen somit mit erheblichem Aufwand hieran angepasst bzw. entsprechend konstruiert werden. Darüber hinaus erweisen sich externe Robotersysteme häufig als umständlich zu justieren und/oder erfüllen nicht den gegebenen Bedarf an einem möglichst kompakten Inkubator mit integrierter Ein- und Ausfuhrmechanik.

Vor diesem Hintergrund ist es die Aufgabe der vorliegenden Erfindung einen möglichst kompakten, einfach zu handhabenden und gegenüber dem Stand der Technik verbesserten Inkubator der eingangs genannten Art mit Ein- und Ausfuhrmechanik zum Be- und Entladen der Probenkammer bereitzustellen, mit welchem die vorstehend erwähnten Nachteile beseitigt werden.

Diese Aufgabe wird mit einem Inkubator nach Anspruch 1 gelöst.

Dabei ist neben den bereits einleitend genannten und gattungsgemäßen Merkmalen vorgesehen, dass die wenigstens eine Ein- und Ausfuhrmechanik vollständig außerhalb der Probenkammer montiert ist und den in die Probenkammer eingefahrenen Probenbehältnisträger auch bei verschlossener Zugangsöffnung trägt.

Mit anderen Worten wird im Rahmen der vorliegenden Erfindung somit ein Inkubator mit einer Ein- und Ausfuhrmechanik zum Be- und Entladen der Probenkammer mit den in geeigneten Probenbehältnissen zu temperierenden Proben bereitgestellt, bei welchem die außerhalb der Probenkammer montierte, also außerhalb der Probenkammer gelagerte bzw. abgestützte Ein- und Ausfuhrmechanik den innerhalb der Probenkammer befindlichen Probenbehältnisträger auch bei geschlossener Zugangsöffnung trägt. Mithin ist die gesamte Halterung (also die Aufhängung bzw. Lagerung) der Ein- und Ausfuhrmechanik des Inkubators außerhalb der Probenkammer angeordnet, also durch wenigstens eine die Probenkammer begrenzende Probenkammerwand, welche vorteilhaft fest innerhalb des Inkubators montiert ist, von dem Innenraum der Probenkammer abgeschottet.

Hierdurch wird einerseits gewährleistet, dass zumindest die zu Montagezwecken erforderliche Halterung der Ein- und Ausfuhrmechanik zu keinem Zeitpunkt der innerhalb der Probenkammer gegebenen Atmosphäre ausgesetzt ist. Ferner ist die Ein- und Ausfuhrmechanik nicht innerhalb der Probenkammer, z.B. an einer die Probenkammer begrenzenden Probenkammerwand, montiert, was auch die Reinigung der Probenkammer wegen einer insoweit besseren Zugänglichkeit deutlich vereinfacht und wodurch sich auch eine zuverlässigere Sterilität des Inkubators erreichen lässt. Und Schließlich wird im Rahmen der vorliegenden Erfindung eine einfach zu handhabende und in kompakter Bauweise realisierbare Ein- und Ausfuhrmechanik bereitgestellt, die den während eines Inkubationsvorgangs innerhalb der Probenkammer befindlichen Probenbehältnisträger dauerhaft, also auch während des Inkubationsvorgangs, trägt, womit kein Absetzen oder Herausziehen des Probenbehältnisträgers vor dem Schließen der Zugangsöffnung der Probenkammer notwendig ist.

Es versteht sich, dass im Rahmen der vorliegenden Erfindung verschiedene Probenbehältnisse und hieran entsprechend angepasste oder anpassbare Probenbehältnisträger Verwendung finden können, wobei - im Sinne der besonders bevorzugten Verwendung standardisierter Probenbehältnisse - insbesondere so genannte Mikrotiterplatten (im Englischen auch "microwell plates" oder "microplates" genannt) und hieran angepasste Probenbehältnisträger Verwendung finden. Bei Mikrotiterplatten handelt es sich um im Wesentlichen rechteckige, meist aus Kunststoff hergestellte Probenbehältnisse mit einer Mehrzahl an separaten - in Reihen und Spalten angeordneten - Kavitäten (engl.: "wells") zur Aufnahme von Proben, die von dem hieran angepassten Probenbehältnisträger entweder vollflächig getragen oder zumindest in einem Randbereich gehalten werden.

Typische Mikrotiterplatten, die im Rahmen der vorliegenden Erfindung vorteilhaft Verwendung finden können, besitzen dabei z.B. 6, 12, 24, 48, 96, 384 oder 1536 einzelne Kavitäten zur Aufnahme von - biologischen und/oder chemischen - Proben, die mittels geeigneter Pipetten (z.B. unter Verwendung eines Pipettierroboters mit Mehrkanalpipetten) in die betreffenden Kavitäten eingebracht werden. Ein erfindungsgemäßer Inkubator kann dabei durch geeignete Dimensionierung der Probenkammer und der Zugangsöffnung auch an die Verwendung so genannter "deep-well microplates", also Mikrotiterplatten mit vergleichsweise tiefen Kavitäten, angepasst sein.

Die Probenkammer eines erfindungsgemäßen Inkubators ist auf geeignete Weise temperierbar, z.B. mittels die Probenkammerwände beheizenden Heiz- und/oder Kühlelementen in Form von Heizfolien, Peltier- und/oder PTC-Elementen, die, wie dies aus dem Stand der Technik bekannt ist, zur Erzielung einer möglichst homogenen Temperatur innerhalb der Probenkammer an geeigneter Stelle innerhalb des Inkubators anzuordnen sind, z.B. an der nicht dem Innenraum der Probenkammer zugewandten Rückseite wenigstens einer die Probenkammer begrenzenden Probenkammerwand. Entsprechende Heiz-/Kühlemente müssen jedoch nicht unmittelbar einer Probenkammerwand anliegen, sondern können vorteilhaft z.B. auch mittels geeigneter (z.B. flach gestalteter) Heat-Pipes (sog. "Vapor Chamber") zur Erzielung einer möglichst unifmormen Temperierung der wenigstens einen Probenkammerwand mit dieser in wärmeleitenden Kontakt gebracht werden, wobei ein Fachmann ersichtlich auch die Geometrie und das Material der Probenkammer in fachüblicher Weise geeignet gestaltet bzw. wählt.

Im Übrigen kann ein erfindungsgemäßer Inkubator neben geeigneten Mitteln zur Temperierung der Probenkammer auch weitere Mittel zur gezielten Einstellung weiterer Umgebungsbedingungen innerhalb der Probenkammer aufweisen, z.B. Mittel zur Einstellung der Luftfeuchte, Mittel zur Zuführung eines oder mehrerer verschiedener Gase, Mittel zur Beaufschlagung der Proben mit elektromagnetischer Strahlung (z.B. infrarotes oder ultraviolettes Licht, Mikrowellen, etc.), um nur einige Beispiele zu nennen.

Ein erste bevorzugte Weiterbildung eines erfindungsgemäßen Inkubators sieht vor, dass die Ein- und Ausfuhrmechanik nicht nur vollständig außerhalb der Probenkammer montiert, sondern - auch bei geschlossener Probenkammer mit innerhalb der Probenkammer befindlichem Probenbehältnisträger - vollständig außerhalb der Probenkammer angeordnet ist. Selbstverständlich muss auch dabei im erfindungsgemäßen Sinn dafür Sorge getragen sein, dass der bei geschlossener Zugangsöffnung innerhalb der Probenkammer angeordnete Probenbehältnisträger auf geeignete Weise - z.B. mittels einer geeigneten Halterung, welche ggfs. einen die Zugangsöffnung zur Probenkammer verschließenden Deckel teilweise durchdringt - von der außerhalb der Probenkammer angeordneten Ein- und Ausfuhrmechanik getragen ist, wobei gleichzeitig der notwendige (thermische) Verschluss der Probenkammer zu gewährleisten ist.

In besonders bevorzugter Weise kann im Rahmen der vorliegenden Erfindung vorgesehen sein, dass die wenigstens eine Zugangsöffnung mittels eines Deckels verschlossen wird, wobei der Deckel vorteilhaft beheiz- und/oder kühlbar ist.

Durch einen beheizbaren bzw. kühlbaren Deckel kann eine besonders homogene Temperaturverteilung der ihrerseits temperierbaren Probenkammer bereitgestellt werden, wobei insbesondere die Beheizbarkeit des Deckels auch einer ansonsten möglichen Kondensatbildung an der zum Innenraum der Probenkammer weisenden Innenseite des die Zugangsöffnung verschließenden Deckels wirksam vorbeugen kann.

In weiterer Fortbildung des vorliegenden Erfindungskonzepts kann vorgesehen sein, dass der wenigstens eine Deckel zum Verschließen einer Zugangsöffnung derart an der wenigstens einen Ein- und Ausfuhrmechanik angeordnet ist, dass der Deckel die Zugangsöffnung bei vollständig in die Probenkammer eingefahrenem Probenbehältnisträger verschließt. Mit anderen Worten trägt die außerhalb der Probenkammer gelagerte bzw. angeordnete Ein- und Ausfuhrmechanik eines erfindungsgemäßen Inkubators vorteilhaft nicht nur den durch die Zugangsöffnung der Probenkammer ein- und ausfahrbaren Probenbehältnisträger, sondern gleichzeitig auch den Deckel, mit welchem besagte Zugangsöffnung vorteilhaft luft- und gasdicht geschlossen wird. Hierdurch wird ersichtlich, dass im Rahmen der vorliegenden Erfindung gleichzeitig realisierbar ist, dass die Ein- und Ausfuhrmechanik gleichzeitig außerhalb der Probenkammer angeordnet und den innerhalb der durch den Deckel verschlossenen Probenkammer angeordneten Probenbehältnisträger tragen kann.

Ferner ist im Rahmen der Erfindung vorgesehen, dass die wenigstens eine Ein- und Ausfuhrmechanik mit zwei Führungen schubladenartig ausgestaltet ist, wobei die zwei Führungen links- und rechtsseitig jeweils außerhalb einer die Probenkammer seitlich begrenzenden Wand auf ungefähr gleicher Höhe angeordnet sind und wobei der Probenbehältnisträger an einem Querträger befestigt ist, welcher zwei in den beiden Führungen geführte Schienen der Ein- und Ausfuhrmechanik miteinander verbindet. Dies erlaubt eine besonders kompakte Bauform eines erfindungsgemäßen Inkubators mit außerhalb der Probenkammer angeordneter Ein- und Ausfuhrmechanik, wobei in diesem Zusammenhang bevorzugt vorgesehen sein kann, dass auch der Deckel an dem Querträger der Ein- und Ausfuhrmechanik befestigt oder daran schwenkbar gelagert ist.

Der Querträger ist dabei vorteilhaft in einem Endbereich der beiden seitlich neben der Probenkammer aus dem Inkubatorgehäuse entsprechend geführt heraus- und hereinfahrbaren Schienen befestigt und vorteilhaft stets horizontal und parallel zu einer Gehäusefront des Inkubators orientiert. Er kann somit in vorteilhafter Weise - inklusive daran befestigtem Deckel und daran gehaltenem Probenbehältnisträger - zwischen einer ersten die Zugangsöffnung freigebenden Stellung und einer zweiten die Zugangsöffnung mittels des Deckels verschließenden Stellung bewegt werden. In der ersten Stellung sollte der Probenbehältnisträger ersichtlich derart weit aus der Probenkammer des Inkubators herausgefahren sein, dass er - entweder manuell oder mittels eines externen Robotersystems - mit einem Probenbehältnis bestückt bzw. ein Probenbehältnis von diesem entnommen werden kann. In der zweiten Stellung ist der Probenbehältnisträger vollständig in die Probenkammer eingefahren, während der ebenfalls am Querträger montierte Deckel die Zugangsöffnung verschließt. Hierbei kann eine den Probenbehältnisträger mit dem Querträger verbindende Halterung auf geeignet abgedichtete Weise zumindest teilweise oder vollständig durch den die Zugangsöffnung verschließenden Deckel hindurchragen bzw. ihrerseits an der zur Probenkammer weisenden Seite des Deckels (und somit mittelbar am Querträger der Ein- und Ausfuhrmechanik) befestigt sein.

In diesem Zusammenhang sei klargestellt, dass bei der vorstehend erläuterten Ausgestaltung eines erfindungsgemäßen Inkubators die gesamte Ein- und Ausfuhrmechanik als vollständig außerhalb der Probenkammer angeordnet anzusehen ist, da der Probenbehältnisträger, die den Probenbehältnisträger am Querträger befestigende Halterung und der Deckel im erfindungsgemäßen Sinne nicht als Teil der eigentlichen Ein- und Ausfuhrmechanik anzusehen sind, selbst wenn besagte Bauteile ggfs. integral mit den weiteren Bestandteilen der Ein- und Ausfuhrmechanik ausgestaltet wären. Die Ein- und Ausfuhrmechanik wird in diesem Ausführungsbeispiel der Erfindung vielmehr durch die vorteilhaft im Inkubatorgehäuse angeordneten Führungen, die darin geführten Schienen und den die Schienen verbindenden Querträger gebildet.

Der Querträger der schubladenartigen Ein- und Ausfuhrmechanik eines erfindungsgemäßen Inkubators der vorstehend beschriebenen Art ist vorteilhaft aus einem Material möglichst geringer Wärmeleitfähigkeit ausgestaltet oder, besonders bevorzugt, zumindest bereichsweise mit einer (thermischen) Isolierung ummantelt.

Ersichtlich ist es zur Realisierung einer automatischen bzw. automatisierbaren Be- und Entladung eines erfindungsgemäßen Inkubators von Vorteil, wenn die (wenigstens eine) Ein- und Ausfuhrmechanik (wenigstens) eine z.B. elektronisch ansteuerbare Antriebseinheit aufweist bzw. von dieser angetrieben ist, wobei die Antriebseinheit vorteilhaft durch einen Elektromotor gebildet sein kann. Eine solche Antriebseinheit kann dann in bevorzugter Weiterbildung der vorliegenden Erfindung (ebenfalls) vollständig außerhalb der Probenkammer angeordnet sein, womit sie einerseits nicht der ggfs. schädlichen Atmosphäre in der Probenkammer und andererseits auch nicht unmittelbar der ggfs. hohen Temperatur in der Probenkammer ausgesetzt ist.

Eine besonders zweckmäßige Ausgestaltung der vorliegenden Erfindung sieht vor, dass die Probenkammer zu fünf Seiten hin durch wenigstens eine innerhalb eines Gehäuses des Inkubators fest angeordnete Probenkammerwand und zur sechsten Seite hin durch einen die wenigstens eine verschließbare Zugangsöffnung aufweisenden Abschlussdeckel begrenzt bzw. gekapselt ist. Dabei ist also die Probenkammer z.B. bodenseitig, deckenseitig, hinterseitig, links- und rechtsseitig, durch fest innerhalb des Inkubators montierte Probenkammerwände begrenzt, wobei ein Teil oder alle der einander angrenzenden Probenkammerwände integral aus einem geeignet geformten bzw. geeignet umgeformten Bauteil ausgestaltet sein können. Nicht einteilig mit benachbarten Probenkammerwänden ausgestaltete Probenkammerwände sind vorteilhaft an benachbarte (Seiten)Wände angeschweißt, angeformt oder zumindest geeignet dagegen abgedichtet. Die sechste Seite der Probenkammer, die vorteilhaft die Frontseite des Inkubators darstellt und in welcher die wenigstens eine Zugangsöffnung zur Probenkammer ausgebildet ist, wird vorteilhaft durch einen - z.B. am Außengehäuse des Inkubators montierten - Abschlussdeckel gebildet, der auf geeignete Weise gegen die ihm angrenzenden Probekammerwände abgedichtet ist. Die wenigstens ein im Abschlussdeckel ausgebildete Zugangsöffnung ist zu Be- und Entladungszwecken der Probenkammer geeignet dimensioniert und mittels eines separaten Deckels, der z.B. an der Ein- und Ausfuhrmechanik montiert sein kann, verschließbar.

Bei dieser Ausgestaltung eines erfindungsgemäßen Inkubators kann dann in nochmals bevorzugter Weise vorgesehen sein, dass die wenigstens eine - die Probenkammer zu fünf Seiten hin begrenzende - Probenkammerwand mit Ausnahme von darin gegebenenfalls vorgesehenen Durchbrechungen zur Zuführung von Gasen in die Probenkammer und/oder zur Durchführung von Sensoren in die Probenkammer keine weiteren Durchbrechungen und/oder keine an der Probenkammerwand oder durch die Probenkammerwand hindurch montierten Anbauelemente aufweist. Ersichtlich wird hierdurch ein Inkubator mit einer besonders einfach zu reinigenden Probenkammer bereitgestellt.

In abermals bevorzugter Weiterbildung dieses Konzepts kann dann vorgesehen sein, dass die wenigstens eine Probenkammerwand auf ihrer die Probenkammer begrenzenden Innenseite einen glatten Verlauf mit Rundungen zwischen den verschiedenen Seiten der Probenkammer hat, was eine nochmals einfachere und zuverlässigere Reinigung der Probenkammer erlaubt.

Von besonderem Vorteil ist im Rahmen der vorliegenden Erfindung, wenn die wenigstens eine Ein- und Ausfuhrmechanik, der von der wenigstens einen Ein- und Ausfuhrmechanik getragene Probenbehältnisträger und das Probenbehältnis in keiner Stellung der Ein- und Ausfuhrmechanik mit der wenigsten einen Probenkammerwand in direktem Kontakt stehen. Dabei wird also der Probenbehältnisträger von der Ein- und Ausfuhrmechanik stets schwebend, d.h. ohne Kontakt zu den im Inkubator fest verbauten Wänden in der Probenkammer, getragen, womit insbesondere eine Mehrzahl an Probenbehältnissen gleichzeitig (und ggfs. übereinander angeordnet) in ein- und derselben Probenkammer temperierbar sind, ohne dass es hierfür spezieller Einbauten in die Probenkammer bedarf.

Weiterhin kann im Rahmen der Erfindung vorgesehen sein, dass die Probenkammer des Inkubators eine Mehrzahl an Zugangsöffnungen und eine Mehrzahl an jeweils einer Zugangsöffnung zugeordneten Ein- und Ausfuhrmechaniken aufweist, so dass ein- und dieselbe Probenkammer des Inkubators zur gleichzeitigen Temperierung der Proben in verschiedenen Probenbehältnissen dienen kann, wobei hierzu jede Ein- und Ausfuhrmechanik vorteilhaft genau einen Probenbehältnisträger trägt und diesen im erfindungsgemäßen Sinn - gemeinsam mit dem jeweils darauf oder damit getragenen Probenbehältnis - durch die jeweilige Zugangsöffnung in die Probenkammer ein- und aus der Probenkammer ausfahren kann.

Dabei kann beispielsweise vorgesehen sein, dass die verschiedenen Zugangsöffnungen, die diesen zugeordneten Ein- und Ausfuhrmechaniken und/oder die von den jeweiligen Ein- und Ausfuhrmechaniken getragenen Probenbehältnisträger sich unterscheiden und an verschiedene Arten von Probenbehältnissen angepasst sind, was jedoch nicht zwingend ist.

Und schließlich ist in einer nochmals bevorzugten Weiterbildung eines solchen Inkubators mit einer Mehrzahl an Zugangsöffnungen und diesen zugeordneten Ein- und Ausfuhrmechaniken (zum Be- und Entladen von Proben durch die jeweilige Zugangsöffnung) vorgesehen, dass die Zugangsöffnungen der Probenkammer in einem die Probenkammer zu einer Seite hin begrenzenden Abschlussdeckel ausgebildet sind, wobei dem Inkubator wenigstens zwei austauschbare Abschlussdeckel mit einer verschiedenen Anzahl an Zugangsöffnungen und/oder einer verschiedenen Größe der Zugangsöffnungen zugeordnet sind.

Hierdurch kann ein Inkubator durch Austausch des Abschlussdeckels an verschiedene Einsatzzwecke angepasst werden, z.B. falls mit diesem verschiedene Arten von Probenbehältnissen temperiert werden sollen. So kann z.B. ein erster von zwei austauschbaren Abschlussdeckeln eine erste Mehrzahl (z.B. zwei, vier, sechs, etc.) an Zugangsöffnungen zur gleichzeitigen Beladung der Probenkammer des Inkubators mit der genannten Mehrzahl an üblichen "microplates" aufweisen, während ein zweiter (Austausch-)Abschlussdeckel z.B. eine geringere Anzahl (z.B. ein, zwei, drei, etc.) an gegenüber den Zugangsöffnungen im ersten Abschlussdeckel größeren Zugangsöffnungen zur Beladung des Inkubators mit gegenüber üblichen "microplates" deutlich höheren "deep-well microplates" aufweist.

Da nach Austausch des ersten durch den zweiten Abschlussdeckel (mit einer geringeren Anzahl an Zugangsöffnungen) für den Betrieb des Inkubators nur mehr eine geringere Anzahl an Ein- und Ausfuhrmechaniken benötigt werden, ist im Rahmen dieses Konzepts von besonderem Vorteil, wenn hierbei zumindest ein Teil der Ein- und Ausfuhrmechaniken - bevorzugt ohne Werkzeugeinsatzderart demontierbar ist, dass nach erfolgter Reduzierung der Anzahl an Zugangsöffnungen nur mehr diejenigen Ein- und Ausfuhrmechaniken funktionsfähig bzw. montiert bleiben, mit denen die (reduzierte Zahl an) verbleibenden Zugangsöffnungen beschickt werden kann.

Bei verschieden großen Zugangsöffnungen in den verschiedenen (Austausch-)Abdeckplatten muss dann ersichtlich ggfs. auch der bzw. die die jeweiligen Zugangsöffnungen verschließenden Deckel an die verschieden großen Zugangsöffnungen anpassbar sein bzw. durch geeignet dimensionierte (Austausch-)Deckel austauschbar sein.

Im Rahmen dieses erfindungszugehörigen Konzepts kann somit insbesondere ein Set aus einem erfindungsgemäßen Inkubator mit zwei verschiedenen (Austausch-)Abschlussdeckeln und ggfs. verschiedenen (Austausch-)Deckeln zum Verschließen der ggfs. verschieden großen Zugangsöffnungen in den verschiedenen Abschlussdeckeln bereitgestellt werden, womit der erfindungsgemäße Inkubator auf einfache Weise an verschiedene Anforderungsprofile anpassbar ist.

Nachfolgend wird ein Ausführungsbeispiel der Erfindung anhand der Zeichnung näher erläutert. Dabei zeigt:
- Fig. 1: eine perspektivische Ansicht eines Ausführungsbeispiels eines erfindungsgemäßen Inkubators mit insgesamt vier Ein- und Ausfuhrmechaniken, von denen sich vorliegend die oberste in einer vollständig ausgefahrenen Stellung befindet,
- Fig. 2: eine perspektivische Ansicht des Inkubators aus Fig. 1 mit vollständig eingefahrenen Einund Ausfuhrmechaniken,
- Fig. 3: einen Teilschnitt durch den Inkubator aus Fig. 2 gemäß dortiger Schnittlinie III-III,
- Fig. 4: eine weitere Darstellung des Inkubators aus Fig. 1 mit zur besseren Übersichtlichkeit ausgeblendetem Abschlussdeckel und zwei fehlenden Ein- und Ausfuhrmechaniken und
- Fig. 5: ein weiteres Ausführungsbeispiel eines erfindungsgemäßen Inkubators mit nur zwei montierten Ein- und Ausfuhrmechaniken und einem gegenüber dem Inkubator aus Fig. 1 abgewandelten Abschlussdeckel.

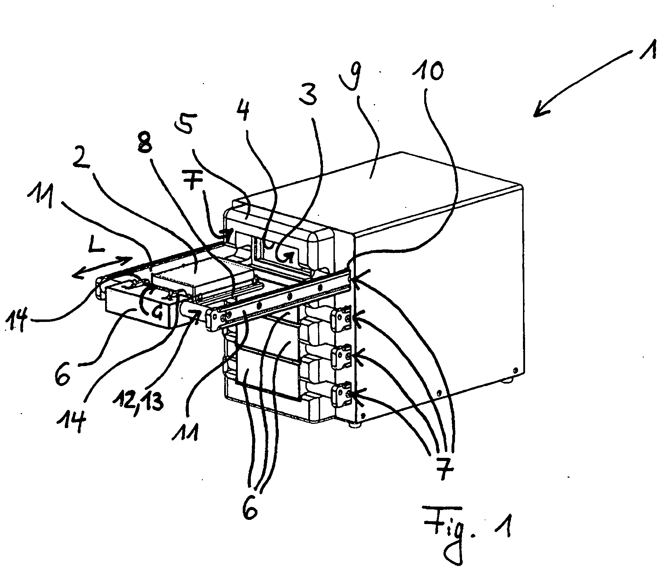

Das in den Fig. 1 - 4 wie vorstehend erläutert in verschiedenen Ansichten dargestellte Ausführungsbeispiel eines erfindungsgemäßen Inkubators 1 dient zur gleichzeitigen Beaufschlagung von in insgesamt vier Probenbehältnissen 2 aufgenommenen Proben mit einer spezifisch vorgebbaren Temperatur. Der Inkubator 1 weist hierzu eine temperierbare Probenkammer 3 auf, welche von einer Frontseite F des Inkubators 1 durch insgesamt vier verschließbare Zugangsöffnungen 4 zugänglich ist.

Die Zugangsöffnungen 4 sind dabei in einer die Probenkammer 3 zur Frontseite F hin begrenzenden Abschlussplatte 5 ausgebildet und werden jeweils mittels hieran geeignet angepasster Deckel 6 dicht verschlossen.

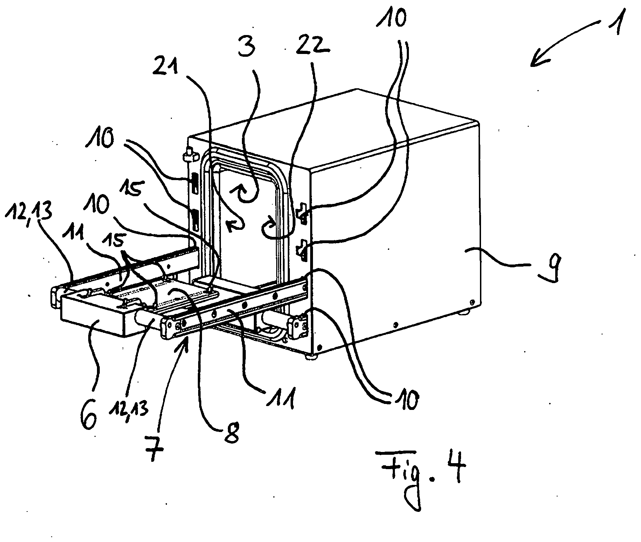

Der Inkubator 1 weist ferner insgesamt vier - schubladenartig gestaltete - Ein- und Ausfuhrmechaniken 7 auf, welche jeweils einen Probenbehältnisträger 8 tragen und mit welchen der Probenbehältnisträger 8 durch die der jeweiligen Ein- und Ausfuhrmechanik 7 zugeordneten Zugangsöffnung 4 in die Probenkammer 3 eingefahren und aus dieser herausgefahren werden kann. Jeder Probenbehältnisträger 8 ist seinerseits dazu eingerichtet ist, je ein Probenbehältnis 2 aufzunehmen bzw. zu tragen. Jede der vorliegend insgesamt vier Ein- und Ausfuhrmechaniken 7 ist vollständig außerhalb der Probenkammer 3 montiert und vollständig außerhalb der Probenkammer 3 angeordnet und umfasst dabei je zwei - auf gleicher Höhe des Inkubators 1 - angeordnete Führungen 10, die links und rechtsseitig außerhalb einer die Probenkammer 3 seitlich begrenzenden Wand 21, 22 (siehe Fig. 4) angeordnet sind. In diesen Führungen 10 ist jeweils eine Schiene 11 gemäß Doppelpfeil L linear beweglich geführt, wobei die zwei Schienen 11 der jeweiligen Ein- und Ausfuhrmechanik 7 in einem aus dem Außengehäuse 9 des Inkubators 1 hervorragenden Endbereich der jeweiligen Schiene 11 mittels eines Querträgers 12 stabil miteinander verbunden sind.

Am Querträger 12 der jeweiligen Ein- und Ausfuhrmechanik 7, welcher bereichsweise mit einer Isolierung 13 ummantelt ist, ist sowohl der die jeweilige Zugangsöffnung 4 im Abschlussdeckel 5 verschließende Deckel 6 als auch der sich vom Querträger 12 in Richtung zur Probenkammer 3 erstreckende Probenbehältnisträger 8 montiert, wobei der Probenbehältnisträger 8 mittels je zweier Halteklammern 14 fest am Querträger 12 befestigt ist.

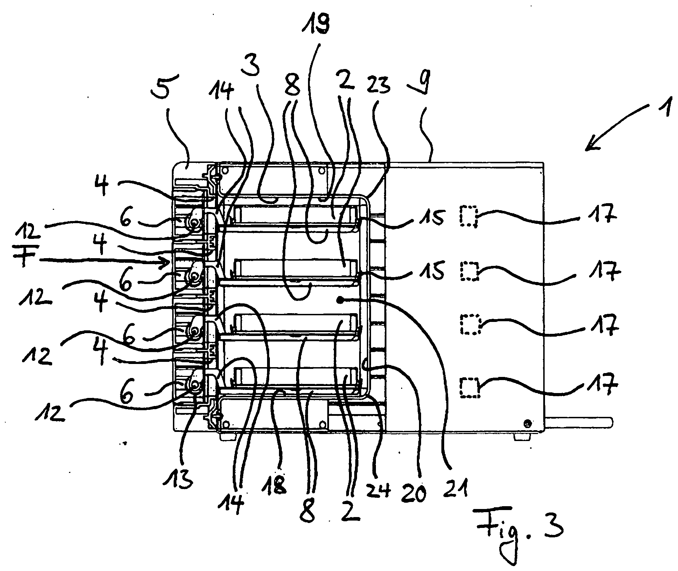

Die Probenbehältnisträger 8 werden, wie dies insbesondere in Fig. 3 gut erkennbar ist, von dem stets außerhalb der Probenkammer 3 befindlichen Querträger 12 der Ein- und Ausfuhrmechanik 7 auch dann gehalten, wenn der Probenbehältnisträger 8 vollständig in die Probenkammer 3 eingefahren und die jeweilige Zugangsöffnung 4 mittels des betreffenden Deckels 6 verschlossen ist.

Um eine stabile Halterung der vorliegend als Mikrotiterplatten bzw. "microplates" ausgestalteten Probenbehältnisse 2 auf bzw. an dem Probenbehältnisträger 8 zu gewährleisten, sind dort unter Federkraft geeignet vorgespannte und in ihrer Lage an die Geometrie des zu verwendenden Probenbehältnisses 2 angepasste bzw. anpassbare Halteelemente 15 vorgesehen.

Die insgesamt vier Deckel 6 sind am Querträger 12 der jeweiligen Ein- und Ausfuhrmechanik 7 gemäß Pfeil C um dessen Längsachse schwenkbar gelagert. Geeignete Anschläge und Führungselemente am Inkubator 1 bzw. an der Ein- und Ausfuhrmechanik 7 des Inkubators 1 stellen sicher, dass der Deckel 6 bei mittels der Ein- und Ausfuhrmechanik 7 vollständig eingefahrenem Probenbehältnisträger 8 die ihm zugeordnete Zugangsöffnung 4 in einer aufrechten Lage vollständig verschließt und dass der Deckel 6 unmittelbar bei Beginn des Ausfahrens des Probenbehältnisträgers 8 aus der Probenkammer 3 um eine durch den Querträger 12 vorgegebene Schwenkachse - vorteilhaft um ca. 90° in eine horizontale Lage - kippt, wie dies in der Darstellung des Inkubators 1 aus Fig. 1 bei der dort obersten, vollständig ausgefahrenen Einund Ausfuhrmechanik 7 gezeigt ist.

Durch das beim Herausfahren der Ein- und Ausfahrmechanik 7 anfänglich erfolgende Verschwenken des Deckels 6 wird das Öffnen der jeweiligen Zugangsöffnung 4 der Probenkammer 3 erleichtert, da der Deckel 6 hierdurch sukzessive von der Zugangsöffnung 4 abgehoben bzw. abgekippt wird, so dass ein ruckartiges Öffnen der Zugangsöffnung 4 zuverlässig vermieden werden kann.

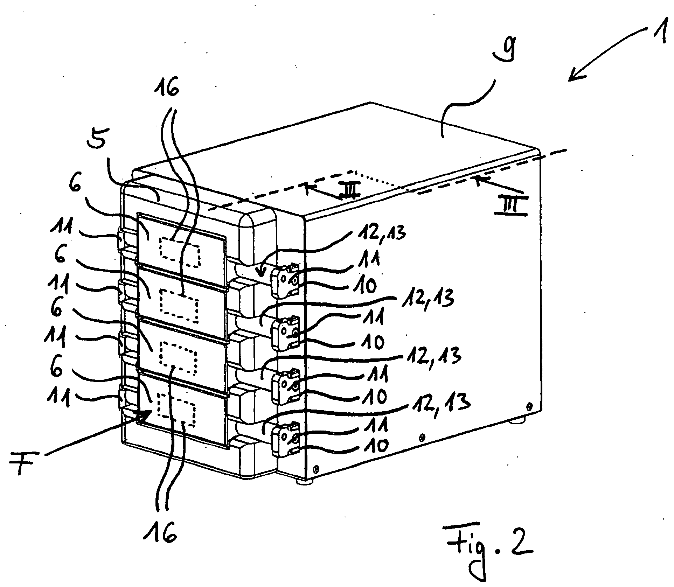

In Fig. 2 ist schematisch angedeutet, dass in die jeweiligen Deckel 6 ein Heiz- und/oder Kühlelement 16, z.B. in Art eines PTC-Elements, verbaut sein kann, womit insbesondere durch eine hierdurch mögliche Beheizung der zur Probenkammer 3 weisenden Innenseite des jeweiligen Deckels 6 eine Bildung von Kondensat an der Deckelinnenseite vermeidbar ist.

Der in Fig. 3 (gemäß der gewinkelt verlaufenden Schnittlinie III-III aus Fig. 2) nicht im Querschnitt dargestellte hintere Teil des Inkubators 1 dient der Aufnahme seiner nicht dargestellten Elektronik sowie der Aufnahme von - gestrichelt dargestellten Antriebseinheiten 17 (z.B. Elektromotoren) - zum automatisiert erfolgenden Antrieb der in den Führungen 10 geführten Schienen 11 der jeweiligen Ein- und Ausfuhrmechanik 7, welche wegen der gegebenen Schnittführung in Fig. 3 nicht gezeigt sind.

Die Probenkammer 3 des Inkubators 1 wird zu fünf Seiten hin, nämlich bodenseitig, deckenseitig, hinterseitig, links- und rechtsseitig durch fest innerhalb des Inkubators 1 verbaute Probenkammerwände 18, 19, 20 21, 22 (siehe Fig. 3 und 4) begrenzt, die im vorliegenden Beispiel allesamt glatt, ohne Durchbrüche und mit Rundungen 23, 24 zwischen den verschiedenen Seiten ausgestaltet sind. Ersichtlich können die verschiedenen Probenkammerwände 18, 19, 20, 21, 22 zumindest teilweise aus ein- und demselben Bauteil hergestellt sein.

In der Darstellung des Inkubators gemäß Fig. 4 (mit ausgeblendetem Abschlussdeckel 5 und zwei ebenfalls nicht dargestellten bzw. teils demontierten Ein- und Ausfuhrmechaniken) ist gut zu erkennen, dass sich im Rahmen der vorliegenden Erfindung ein Inkubator 1 mit wenigstens einer integrierten Ein- und Ausfuhrmechanik 7 realisieren lässt, dessen Probenkammer 3 wegen der außerhalb der Probenkammer 3 erfolgenden Montage und Anordnung der (wenigstens einen) Ein- und Ausfuhrmechanik 7, wie vorstehend beschrieben, besonders einfach zu reinigen ist.

Sofern die in den Führungen 10 geführten Schienen 11 der jeweiligen Auszugsmechanik 7 und die die Probenkammer 3 zur Frontseite hin begrenzende Abschlussplatte 5 einfach, insbesondere ohne Werkzeugeinsatz, demontierbar sind, ist die - einen vollkommen glatten Verlauf und vorteilhaft kein Durchbrüche aufweisende - Probenkammer 3 besonders einfach zu Reinigungszwecken zugänglich.

Es versteht sich von selbst, dass ein erfindungsgemäßer Inkubator 1 nicht zwingend genau vier, sondern auch eine größere oder kleinere Zahl, insbesondere auch nur eine mittels einer entsprechenden Ein- und Ausfuhrmechanik 7 mit Proben beschickbare Zugangsöffnung 4 zur Probenkammer 3 aufweisen kann.

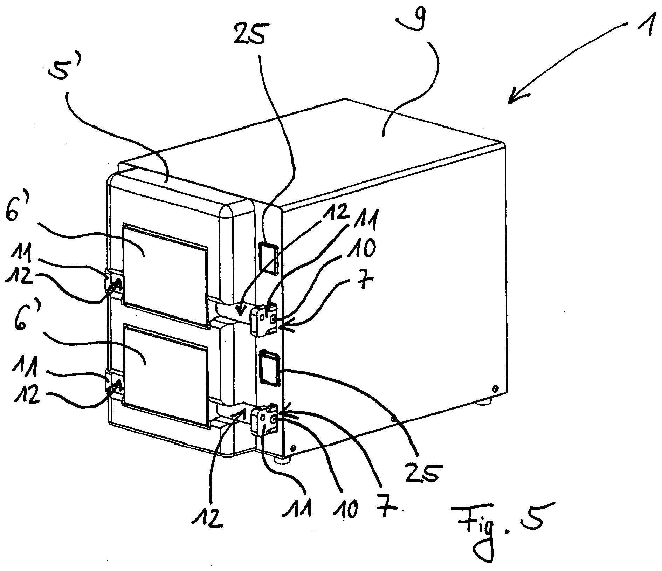

Fig. 5 zeigt in diesem Zusammenhang einen Inkubator 1', welcher im Wesentlichen baugleich zu dem Inkubator 1 aus den Fig. 1 - 4 ist. Er unterscheidet sich hiervon nur dadurch, dass er eine anders gestaltete (Austausch-) Abschlussplatte 5' zur frontseitigen Begrenzung der Probenkammer 3 mit einer geringeren Anzahl, jedoch größeren Zugangsöffnungen und entsprechend hieran angepassten (Austausch-)Deckeln 6' aufweist, die sich zur Ein- und Ausfuhr von insgesamt zwei "deep-well microplates" eignen. Ferner werden bei dem Inkubator 1' aus Fig. 5 nur zwei der eigentlich vier Ein- und Ausfuhrmechaniken 7 benötigt, so dass die nicht benötigten zwei Ein- und Ausfuhrmechaniken 7 durch Entnahme der jeweiligen Schienen 11 aus den betreffenden Führungen 10 teilweise demontiert wurden. Die dabei im Außengehäuse 9 des Inkubators 1 verbleibenden Öffnungen der nicht benötigten Führungen 10 sind mittels entsprechender Deckel 25 abgedeckt, um ein Eindringen von Schmutz in die - außerhalb der Probenkammer 3 gelegenen - Führungen 10 zu verhindern.

## Patentansprüche

1. Inkubator (1) zur Beaufschlagung von in Probenbehältnissen (2) aufgenommenen Proben mit einer spezifisch vorgebbaren Temperatur umfassend
- eine temperierbare Probenkammer (3) mit wenigstens einer verschließbaren Zugangsöffnung (4) und
- wenigstens eine einen Probenbehältnisträger (8) tragende Ein- und Ausfuhrmechanik (7), mit welcher der seinerseits ein Probenbehältnis (2) tragende Probenbehältnisträger (8) durch eine Zugangsöffnung (4) des Inkubators (1) in die Probenkammer (3) eingefahren und aus dieser herausgefahren werden kann,
**dadurch gekennzeichnet,**
**dass** die wenigstens eine Ein- und Ausfuhrmechanik (7) mit zwei Führungen (10) schubladenartig ausgestaltet ist, wobei die zwei Führungen (10) links- und rechtsseitig jeweils außerhalb einer die Probenkammer (3) seitlich begrenzenden Wand (21, 22) auf ungefähr gleicher Höhe angeordnet sind und wobei der Probenbehältnisträger (8) an einem Querträger (12) befestigt ist, welcher zwei in den beiden Führungen (10) geführte Schienen (11) der Ein- und Ausfuhrmechanik (7) miteinander verbindet, und
**dass** die wenigstens eine Ein- und Ausfuhrmechanik (7) vollständig außerhalb der Probenkammer (3) montiert ist und den in die Probenkammer (3) eingefahrenen Probenbehältnisträger (8) auch bei verschlossener Zugangsöffnung (4) trägt.

2. Inkubator nach Anspruch 1,
**dadurch gekennzeichnet,**
**dass** die wenigstens eine Ein- und Ausfuhrmechanik (7) vollständig außerhalb der Probenkammer (3) angeordnet ist.

3. Inkubator nach einem der Ansprüche 1 oder 2,
**dadurch gekennzeichnet,**
**dass** die wenigstens eine Zugangsöffnung (4) mittels eines Deckels (6) verschlossen wird, wobei der Deckel (6) vorteilhaft beheiz- und/oder kühlbar ist.

4. Inkubator nach Anspruch 3,
**dadurch gekennzeichnet,**
**dass** der wenigstens eine Deckel (6) zum Verschließen einer Zugangsöffnung (4) derart an der wenigstens einen Ein- und Ausfuhrmechanik (7) angeordnet ist, dass der Deckel (6) die Zugangsöffnung (4) bei vollständig in die Probenkammer (3) eingefahrenem Probenbehältnisträger (8) verschließt.

5. Inkubator nach Anspruch 1 und Anspruch 4,
**dadurch gekennzeichnet,**
**dass** der Deckel (6) an dem Querträger (12) der Ein- und Ausfuhrmechanik (7) befestigt oder daran schwenkbar gelagert ist.

6. Inkubator nach einem der Ansprüche 1 oder 5,
**dadurch gekennzeichnet,**
**dass** der Querträger (12) zumindest bereichsweise mit einer Isolierung (13) ummantelt ist.

7. Inkubator nach einem der vorangehenden Ansprüche,
**dadurch gekennzeichnet,**
**dass** wenigstens eine die wenigstens eine Ein- und Ausfuhrmechanik (7) antreibende Antriebseinheit (17) vollständig außerhalb der Probenkammer angeordnet ist.

8. Inkubator nach einem der vorangehenden Ansprüche,
**dadurch gekennzeichnet,**
**dass** die Probenkammer (3) zu fünf Seiten hin durch wenigstens eine innerhalb eines Gehäuses (9) des Inkubators (1) fest angeordnete Probenkammerwand (18, 19, 20, 21, 22) und zur sechsten Seite hin durch einen die wenigstens eine verschließbare Zugangsöffnung (4) aufweisenden Abschlussdeckel (5, 5') begrenzt ist.

9. Inkubator nach Anspruch 8,
**dadurch gekennzeichnet,**
**dass** die wenigstens eine Probenkammerwand (18, 19, 20, 21, 22) mit Ausnahme von darin gegebenenfalls vorgesehenen Durchbrechungen zur Zuführung von Gasen in die Probenkammer (3) und/oder zur Durchführung von Sensoren in die Probenkammer (3) keine weiteren Durchbrechungen und/oder keine an der Probenkammerwand (18, 19, 20, 21, 22) oder durch die Probenkammerwand (18, 19, 20, 21, 22) hindurch montierten Anbauelemente aufweist.

10. Inkubator nach Anspruch 8 oder 9,
**dadurch gekennzeichnet,**
**dass** die wenigstens eine Probenkammerwand (18, 19, 20, 21, 22) auf ihrer die Probenkammer (3) begrenzenden Innenseite einen glatten Verlauf mit Rundungen (23, 24) zwischen den verschiedenen Seiten der Probenkammer (3) hat.

11. Inkubator nach einem der vorangehenden Ansprüche,
**dadurch gekennzeichnet,**
**dass** die wenigstens eine Ein- und Ausfuhrmechanik (7), der von der wenigstens einen Ein- und Ausfuhrmechanik (7) getragene Probenbehältnisträger (8) und das Probenbehältnis (2) in keiner Stellung der Ein- und Ausfuhrmechanik (7) mit der wenigsten einen Probenkammerwand (18, 19, 20, 21, 22) in direktem Kontakt stehen.

12. Inkubator nach einem der vorangehenden Ansprüche,
**dadurch gekennzeichnet,**
**dass** die Probenkammer (3) des Inkubators (1) eine Mehrzahl an Zugangsöffnungen (4) und eine Mehrzahl an jeweils einer Zugangsöffnung (4) zugeordneten Ein- und Ausfuhrmechaniken (7) aufweist.

13. Inkubator nach Anspruch 12,
**dadurch gekennzeichnet,**
**dass** die Zugangsöffnungen (4) der Probenkammer (3) in einem die Probenkammer (3) zu einer Seite hin begrenzenden Abschlussdeckel (5) ausgebildet sind, wobei einem Inkubator (1) wenigstens zwei austauschbare Abschlussdeckel (5, 5') mit einer verschiedenen Anzahl an Zugangsöffnungen (4) und/oder einer verschiedenen Größe der Zugangsöffnungen (4) zugeordnet sind.

## Claims

1. An incubator (1) for exposing samples received in sample containers (2) to a temperature that can be predefined in a specific manner, said incubator comprising
- a sample chamber (3), the temperature of which can be controlled and which has at least one closable access opening (4), and
- at least one loading and unloading mechanism (7), which supports a sample container carrier (8) and by means of which a sample container carrier (8) supporting a sample container (2) can be loaded into the sample chamber (3) and unloaded therefrom through an access opening (4) of the incubator (1),
**characterised in that**
the at least one loading and unloading mechanism (7) is formed in a drawer-like manner with two guides (10), wherein the two guides (10) are arranged at approximately the same height, one on the left side and one on the right side, in each case outside a wall (21, 22) delimiting the sample chamber (3) laterally, and wherein the sample container carrier (8) is secured to a crossmember (12) which connects two rails (11) of the loading and unloading mechanism (7), which rails are guided in the two guides (10), and
**in that** the at least one loading and unloading mechanism (7) is mounted completely outside the sample chamber (3) and supports the sample container carrier (8) loaded into the sample chamber (3) also when the access opening (4) is closed.

2. The incubator according to claim 1,
**characterised in that**
the at least one loading and unloading mechanism (7) is arranged fully outside the sample chamber (3).

3. The incubator according to either one of claims 1 or 2,
**characterised in that**
the at least one access opening (4) is closed by means of a cover (6), wherein the cover (6) advantageously can be heated and/or can be cooled.

4. The incubator according to claim 3,
**characterised in that**
the at least one cover (6) for closing an access opening (4) is arranged on the at least one loading and unloading mechanism (7) in such a way that the cover (6) closes the access opening (4) when the sample container carrier (8) is loaded fully into the sample chamber (3).

5. The incubator according to claim 1 and claim 4,
**characterised in that**
the cover (6) is fastened to the crossmember (12) of the loading and unloading mechanism (7) or is mounted pivotably thereon.

6. The incubator according to any one of claims 1 or 5,
**characterised in that**
the crossmember (12) is covered at least in some regions by an insulation (13).

7. The incubator according to any one of the preceding claims,
**characterised in that**
at least one drive unit (17) driving the at least one loading and unloading mechanism (7) is arranged fully outside the sample chamber.

8. The incubator according to any one of the preceding claims,
**characterised in that**
the sample chamber (3) is delimited on five sides by at least one sample chamber wall (18, 19, 20, 21, 22) arranged fixedly within a housing (9) of the incubator (1) and on the sixth side by a closure cover (5, 5') having at least one closable access opening (4).

9. The incubator according to claim 8,
**characterised in that**
the at least one sample chamber wall (18, 19, 20, 21, 22), with the exception of any apertures optionally provided therein for feeding gases into the sample chamber (3) and/or for guiding through sensors into the sample chamber (3), has no further apertures and/or no add-on elements mounted on the sample chamber wall (18, 19, 20, 21, 22) or through the sample chamber wall (18, 19, 20, 21, 22).

10. The incubator according to claim 8 or 9,
**characterised in that**
the at least one sample chamber wall (18, 19, 20, 21, 21, 22) has, on its inner side delimiting the sample chamber (3), a smooth profile with rounded portions (23, 24) between the different sides of the sample chamber (3).

11. The incubator according to any one of the preceding claims,
**characterised in that**
the at least one loading and unloading mechanism (7), the sample container carrier (8) supported by the at least one loading and unloading mechanism (7), and the sample container (2) are not in direct contact with the at least one sample chamber wall (18, 19, 20, 21, 22) in any position of the loading and unloading mechanism (7) .

12. The incubator according to any one of the preceding claims,
**characterised in that**
the sample chamber (3) of the incubator (1) has a plurality of access openings (4) and a plurality of loading and unloading mechanisms (7) associated with an access opening (4) each.

13. The incubator according to claim 12,
**characterised in that**
the access openings (4) of the sample chamber (3) are formed in a closure cover (5) delimiting the sample chamber (3) on one side, wherein an incubator (1) is associated with at least two exchangeable closure covers (5, 5') having a different number of access openings (4) and/or a different size of the access openings (4).

## Revendications

1. Incubateur (1) qui permet d'exposer des échantillons, contenus dans des récipients d'échantillon (2), à une température pouvant être spécifiquement définie au préalable et qui comprend
- une chambre à échantillons (3) thermorégulable comportant au moins une ouverture d'accès (4) pouvant être obturée, et
- au moins une mécanique d'entrée et de sortie (7) qui est pourvue d'un support de récipient d'échantillon (8) et qui permet de faire entrer le support de récipient d'échantillon (8), lequel est pourvu à son tour du récipient d'échantillon (2), dans la chambre à échantillons (3) et de le faire sortir de celle-ci, en passant une ouverture d'accès (4) de l'incubateur (1),
**caractérisé en ce que**
l'au moins une mécanique d'entrée et de sortie (7) est réalisée de manière à comporter deux guidages (10) et à ressembler à un tiroir, les deux guidages (10) étant disposés, approximativement à des hauteurs égales, sur les côtés gauche et droit chacun à l'extérieur d'une paroi (21, 22) délimitant la chambre à échantillons (3) latéralement, et le support de récipient d'échantillon (8) étant fixé sur un support transversal (12) qui relie entre eux deux rails (11) de la mécanique d'entrée et de sortie (7) lesquels sont guidés dans les deux guidages (10), et
que l'au moins une mécanique d'entrée et de sortie (7) est montée intégralement à l'extérieur de la chambre à échantillons (3) tout en étant pourvue du support de récipient d'échantillon (8) rentré dans la chambre à échantillons (3) même lorsque l'ouverture d'accès est fermée (4).

2. Incubateur selon la revendication 1,
**caractérisé en ce que**
l'au moins une mécanique d'entrée et de sortie (7) est disposée intégralement à l'extérieur de la chambre à échantillons (3).

3. Incubateur selon l'une des revendications 1 ou 2,
**caractérisé en ce que**
l'au moins une ouverture d'accès (4) est fermée au moyen d'un couvercle (6), le couvercle (6) pouvant avantageusement être chauffé et/ou refroidi.

4. Incubateur selon la revendication 3,
**caractérisé en ce que**
l'au moins un couvercle (6) destiné à fermer l'ouverture d'accès (4) est disposé sur l'au moins une mécanique d'entrée et de sortie (7) de manière à ce que le couvercle (6) ferme l'ouverture d'accès (4) lorsque le support de récipient d'échantillon (8) est complètement rentré dans la chambre à échantillons (3).

5. Incubateur selon la revendication 1 et la revendication 4,
**caractérisé en ce que**
le couvercle (6) est fixé sur le support transversal (12) de la mécanique d'entrée et de sortie (7) ou monté pivotant sur celle-ci.

6. Incubateur selon l'une des revendications 1 ou 5,
**caractérisé en ce que**
le support transversal (12) est entouré, au moins sur certaines parties, d'une isolation (13).

7. Incubateur selon l'une des revendications précédentes,
**caractérisé en ce qu'**
au moins une unité d'entraînement (17) qui entraîne l'au moins une mécanique d'entrée et de sortie (7) est disposée intégralement à l'extérieur de ladite chambre à échantillons.

8. Incubateur selon l'une des revendications précédentes,
**caractérisé en ce que**
la chambre à échantillons (3) est limitée, sur cinq de ses faces, par au moins une paroi de chambre à échantillons (18, 19, 20, 21, 22) disposée de manière fixe au sein d'un boîtier (9) de l'incubateur (1), et sur sa sixième face par un couvercle de fermeture (5, 5') présentant l'au moins une ouverture d'accès (4) pouvant être fermée.

9. Incubateur selon la revendication 8,
**caractérisé en ce que**
l'au moins une paroi de chambre à échantillons (18, 19, 20, 21, 22) ne présente, à l'exception des percées y étant éventuellement prévues pour introduire des gaz dans la chambre à échantillons (3) et/ou pour faire passer des capteurs dans la chambre à échantillons (3), pas d'autres percées ni d'éléments rapportés qui sont montés sur la paroi de chambre à échantillons (18, 19, 20, 21, 22) ou à travers la paroi de chambre à échantillons (18, 19, 20, 21, 22).

10. Incubateur selon les revendications 8 ou 9,
**caractérisé en ce que**
l'au moins une paroi de chambre à échantillons (18, 19, 20, 21, 22) suit, sur sa face intérieure délimitant la chambre à échantillons (3), un tracé lisse avec des parties arrondies (23, 24) entre les différentes faces de la chambre à échantillons (3).

11. Incubateur selon l'une des revendications précédentes,
**caractérisé en ce que**
ni l'au moins une mécanique d'entrée et de sortie (7), ni le support de récipient d'échantillon (8) dont l'au moins une mécanique d'entrée et de sortie (7) est pourvue ni le récipient d'échantillon (2) ne soient en contact direct avec l'au moins une paroi de chambre à échantillons (18, 19, 20, 21, 22) quelle que soit la position de la mécanique d'entrée et de sortie (7).

12. Incubateur selon l'une des revendications précédentes,
**caractérisé en ce que**
la chambre à échantillons (3) de l'incubateur (1) présente une pluralité d'ouvertures d'accès (4) et une pluralité de mécaniques d'entrée et de sortie (7) dont chacune est associée à une ouverture d'accès (4).

13. Incubateur selon la revendication 12,
**caractérisé en ce que**
les ouvertures d'accès (4) de la chambre à échantillons (3) sont réalisées dans un couvercle de fermeture (5) délimitant la chambre à échantillons (3) sur l'une de ses faces, au moins deux couvercles de fermeture échangeables (5, 5') lesquels comportent un nombre différent d'ouvertures d'accès (4) et/ou des ouvertures d'accès (4) de tailles différentes étant associés à un incubateur (1).
